# FASCICULE DE BREVET EUROPEEN

(11) **EP 0 780 399 B1**
(45) Date de publication et mention de la délivrance du brevet: **20.06.2001**
(21) Numéro de dépôt: 96402824.5
(22) Date de dépôt: 19.12.1996
(51) Int. Cl.: C07H 15/04, C08B 31/18

(54) **Glucuronyl-arabinarates, leur procédé d'obtention et applications de ces produits**
Glucuronylarabinarate, Verfahren zu ihrer Herstellung und Anwendungen dieser Produkte
Glucuronylarabinarates, process for their preparation and uses of these products

(30) Priorité: 21.12.1995 FR 9515268
(43) Date de publication de la demande: 25.06.1997
(73) Titulaire: Roquette Frères, 62136 Lestrem (FR)
(72) Inventeur: Fleche, Guy, 59190 Hazebrouck (FR)
(74) Mandataire: Boulinguiez, Didier

(56) Documents cités:
- WO-A-94/28030
- WO-A-95/07303
- DE-C- 618 164
- FR-A- 2 722 200
- US-A- 4 125 559
- US-A- 4 985 553
- US-A- 5 618 675
- HENDRIKS, KUSTER & MARIN: "The alkaline anthraquinone-2-sulfonate-H2O2-catalyzed oxidative degradation of lactose: An improved Spengler-Pfannenstiel oxidation", CARBOHYDRATE RESEARCH, AMSTERDAM, 1991, Vol. 214, no. , pages 71 à 85
- KIEBOOM & BEKKUM: "Chemical conversion of starch-based glucose syrups", FOOD SCIENCE AND TECHNOLOGY, NEW YORK, 1985, Vol. 14, no. , pages 263 à 334

## Description

La présente invention a pour objet, en tant que produits industriels nouveaux, les glucuronyl-arabinarates.

Elle vise également un procédé d'obtention de ces produits ainsi que leur application en tant qu'agents séquestrants et dispersants dans les compositions lessivielles.

D'autres caractéristiques et avantages de la présente invention apparaîtront à la lecture de la description qui suit.

Par le terme de glucuronyl-arabinarates, on désigne, dans la présente invention, non seulement l'acide glucuronyl (α, 1-3) arabinarique (ou ses sels), mais également les polyglucuronyl-arabinarates, qui sont des molécules de nature polymérique constituées essentiellement d'une chaîne de motifs anhydroglucuroniques reliés par une liaison osidique (α, 1-4) et liés de façon covalente par le même type de liaison (α, 1-3) à une unité terminale d'acide arabinarique.

On dira par la suite que l'acide glucuronyl (α, 1-3) arabinarique, composé d'un motif glucuronyl et d'une unité terminale d'acide arabinarique, possède un DP (degré de polymérisation) de 2. Un acide glucuronyl-arabinarique composé de deux motifs glucuronyl possédera un DP de 3, et ainsi de suite.

Dans les produits de l'invention, ces motifs glucuronyl, dits aussi anhydroglucuroniques, pourront alterner de façon plus ou moins fréquente et plus ou moins répétitive, avec des motifs anhydroglucosidiques, dits également glucosyl, c'est-à-dire non oxydés.

On sait que l'on peut obtenir des produits dont la structure ressemble à ceux de la présente invention : les polyglucuronyl-glucarates, par action de l'acide nitrique sur l'amidon à froid (Food. Sci. Technol. 1985, vol 14, chap 9, page 286, KIEBOOM et VAN BEKKUM). Les polymères en question comportent alors environ un motif anhydroglucuronyl pour un motif anhydroglucosyl et à la différence des produits de l'invention, leur unité terminale est constituée par un acide glucarique.

D'autres polyglucuronyl-glucarates contenant davantage de motifs anhydroglucuronyl par rapport aux motifs anhydroglucosyl, peuvent être obtenus selon les enseignements de la demande internationale de brevet WO 94/28030 par action du dioxyde ou du tétroxyde d'azote sur des amidons ou fécules.

Les polyglucuronyl-glucarates en question peuvent comporter de 70 à 95 % de motifs anhydroglucuronyl pour 5 à 30 % de motifs anhydroglucosyl.

D'autres polyglucuronyl-glucarates contenant au moins 90 % de motifs anhydroglucuronyl pour 10 % de motifs anhydroglucosyl peuvent être obtenus par le procédé décrit dans la demande mondiale de brevet WO 95/07303. Le procédé décrit dans cette demande de brevet consiste à oxyder des hydrolysats d'amidon par action d'hypohalites en présence de quantités catalytiques d'un nitroxyl alcyle binaire ou tertiaire tel que la 1-oxyl-2,2,6,6- tétraméthylpipéridine. Ce procédé s'avère toutefois incapable d'oxyder les oligosaccharides relativement lourds (DP supérieur à 15) en leur fonction hémiacétalique ou cétonique terminale. Ceci se traduit par le fait qu'une quantité substantielle de polyglucuronyl-glucuronates de DP élevé persistent au sein de polyglucuronyl-glucarates de DP plus faible et que les produits obtenus ne sont pas stables en milieu alcalin ou à la chaleur.

Des glucosyl-arabinonates, produits dont la structure ressemble également à celle des produits conformes à la présente invention, peuvent être obtenus par dégradation alcaline oxydative de divers disaccharides à l'aide d'air ou d'oxygène.

Dans ces conditions, ainsi qu'il est expliqué dans le brevet allemand DE 618.164, le maltose fournit le glucosyl (α, 1-3) arabinonate ou, ainsi qu'il est expliqué dans le brevet américain US 4.618.675, le palatinose fournit le glucosyl (α, 1-5) arabinonate.

On aura compris que ces produits diffèrent de la présente invention par le fait que leur unité terminale est un acide arabinonique et non un acide arabinarique. Ils en différent aussi par le fait que l'autre unité constitutive de la molécule est obligatoirement un radical anhydroglucosyl et non un radical anhydroglucuronyl. Toutefois, des produits selon l'invention pourront aussi contenir des motifs anhydroglucosidiques dans la mesure où ils contiennent aussi au moins un motif anhydroglucuronyl et une extrémité terminale d'acide arabinarique.

D'autres produits enfin, dont la structure ressemble aussi à celle des produits de la présente invention peuvent être obtenus par l'oxydation catalytique d'hydrolysats d'amidon à l'aide d'oxygène de l'air et de catalyseurs à métal noble fixé sur charbon, ainsi qu'il est enseigné dans le brevet US 4,985,553 dont la Société Demanderesse est cessionnaire.

Les produits obtenus par ce procédé sont des polyglucosyl-gluconates.

Tous ces glucuronyl-glucarates, glucosyl-arabinonates et glucosyl-gluconates de l'art antérieur sont porteurs de fonctions carboxyliques et, à des degrés divers mais au même titre que d'autres acides carboxyliques tels que l'acide citrique, l'acide gluconique ou les acides polyacryliques, présentent des propriétés séquestrantes qui permettent à ces produits de jouer le rôle de "builders" ou de "cobuilders" dans les formulations lessivielles.

Cependant, aucun d'entre eux ne réunit à lui seul et à un degré suffisant toutes les qualités suivantes :
- pouvoir séquestrant élevé,
- pouvoir dispersant élevé,
- biodégradabilité élevée,
- stabilité élevée,
- prix attractif,
qui sont celles que l'on prête à un "builder" ou "cobuilder" idéal.

En outre, la tendance étant à ce que les compositions lessivielles deviennent de plus en plus concentrées en produits réellement actifs : tensio-actifs, enzymes, agents de blanchiment, il reste de moins en moins de place pour le système "builder-cobuilder" dont le rôle essentiel se réduit après tout à ne corriger que les imperfections des eaux de lavage et notamment leur teneur en sels alcalino-terreux.

Il existait donc un besoin de mettre au point un produit pouvant servir de "builder" ou de "cobuilder" dans les compositions lessivielles modernes et qui possède simultanément toutes les qualités évoquées plus haut.

La Société Demanderesse a trouvé qu'un tel besoin pouvait être satisfait par les glucuronyl-arabinarates de l'invention.

Au terme de nombreux essais, la Société Demanderesse a démontré :
- qu'il était possible de fabriquer industriellement de tels produits avec des matières premières et des procédés peu coûteux ;
- que les pouvoirs séquestrant et dispersant de ces produits étaient d'un niveau tel que leur emploi dans les lessives se traduisait par des taux de redéposition et des taux d'incrustation très faibles des étoffes lavées avec ces lessives ;
- que de tels produits étaient aisément biodégradables ;
- que de tels produits étaient suffisamment stables pour supporter à la fois les contraintes thermiques liées aux procédés de séchage des compositions lessivielles et les contraintes chimiques liées à la stabilité de molécules organiques dans ces compositions lessivielles fortement alcalines.

A ce dernier sujet, l'inconvénient majeur des compositions lessivielles d'hydrolysats d'amidon oxydés selon les techniques antérieures, qu'il s'agisse de glucuronyl-glucarates, de glucosyl-arabinonates ou de glucosyl-gluconates, est la persistance, dans ces hydrolysats d'amidon oxydés, d'extrémités hémiacétaliques réductrices instables à la chaleur et en milieu alcalin.

Un tel défaut est évidemment rédhibitoire et se révèle par une coloration jaune-brun des poudres lessivielles ou des lessives liquides, au mieux après une courte durée de stockage, au pire, dès leur formulation.

Un procédé particulier d'obtention des glucuronyl-arabinarates selon l'invention permet, comme on le verra plus loin, d'obtenir des produits ne présentant pas ce défaut.

En tout premier lieu, la présente invention concerne donc, en tant que produits nouveaux, des glucuronyl-arabinarates.

De préférence, elle concerne des compositions de glucuronyl-arabinarates ayant un degré de polymérisation moyen supérieur ou égal à 2.

De façon plus préférentielle, les produits de l'invention ont un degré de polymérisation moyen compris entre 2 et 50 et de façon encore plus préférentielle compris entre 2 et 10.

Ceci s'explique par le fait que les compositions de glucuronyl-arabinarates selon l'invention ayant les DP moyens préférés développent les pouvoirs séquestrants les plus élevés.

Selon la présente invention, les compositions de glucuronyl-arabinarates préférées sont celles qui contiennent de 100 % à 50 % de motifs anhydroglucuronyl, de préférence de 99 % à 60 % de motifs anhydroglucuronyl, ce pourcentage de motifs anhydroglucuronyl étant exprimé par rapport à la somme des motifs anhydroglucuronyl et anhydroglucosyl.

Ces préférences s'expliquent par le fait qu'il est d'une part difficile d'oxyder de façon complète toutes les fonctions alcools primaires d'un polyglucanne, quel qu'il soit, et d'autre part par le fait qu'en deçà des seuils indiqués, les propriétés séquestrantes et dispersantes des glucuronyl-arabinarates ne se manifestent plus de manière aussi satisfaisante dans l'utilisation de ces produits dans les formulations lessivielles.

Quoi qu'il en soit, les compositions selon l'invention contiennent moins de 1 %, de préférence moins de 0,7 %, et encore plus préférentiellement moins de 0,5 % de sucres réducteurs libres, exprimés en équivalents de glucose libre et mesurés par la méthode de BERTRAND.

La raison de ces préférences tient en ce qu'au-delà des limites indiquées, ces sucres réducteurs libres, qui révèlent la présence d'extrémités hémiacétaliques, se montreraient la cause de phénomènes trop marqués d'instabilité à la chaleur et à l'alcalinité des compositions lessivielles dans lesquelles entreraient les produits de l'invention.

Selon un autre aspect de la présente invention, un procédé d'obtention des glucuronyl-arabinarates conformes à l'invention consiste :
- dans une première étape, à procéder à la dégradation alcaline oxydative d'un hydrolysat d'amidon de manière à obtenir des glucosyl-arabinonates,
- dans une deuxième étape, à procéder à l'oxydation sélective de tout ou partie des fonctions alcools primaires de ces glucosyl-arabinonates.

Dans la présente invention le terme de glucosyl-arabinonates englobe le glucosyl (α, 1-3) arabinonate mais aussi les polyglucosyl-arabinonates, c'est-à-dire des molécules constituées d'une chaîne d'au moins deux motifs anhydroglucosidiques liée de façon covalente à une molécule terminale d'acide arabinonique.

Par hydrolysat d'amidon, la Société Demanderesse veut désigner ici tous les types d'amidon ou de fécule qui ont subi l'action d'acides ou d'enzymes ou des deux, de manière à en obtenir la solubilité dans l'eau et la réduction de la masse moléculaire. Les dextrines, maltodextrines, sirops de glucose, sirops de maltose sont donc concernés et plus particulièrement ceux dont le degré de polymérisation moyen correspond au degré de polymérisation moyen des produits de l'invention qui sont préférés.

Par dégradation alcaline oxydative, on désigne les procédés qui consistent à soumettre des solutions aqueuses de composés oxydables à l'action de l'air ou de l'oxygène finement divisé en milieu fortement alcalin. Cette oxydation peut être catalysée par divers promoteurs, par exemple le bleu de méthylène comme indiqué dans le brevet américain US 2,587,906 ou par un couple redox constitué d'acide anthraquinone-2-monosulfonique et de peroxyde d'hydrogène (HENDRICKS, KUSTER et MARIN, Carb. res, 214 (1991) 71-85). Cette oxydation peut se dérouler à pression atmosphérique à l'aide d'air, ou sous pression à l'aide d'oxygène comme indiqué dans le brevet américain US 4,125,559.

Quoique ces réactions de dégradation alcaline oxydative des sucres réducteurs aient toujours été conduites sur des mono - ou disaccharides tels que notamment le glucose, le mannose, le fructose, le maltose ou le lactose, la Société Demanderesse a remarqué que l'enseignement des documents cités ci-dessus pouvait aussi être étendu aux polysaccharides dotés d'un pouvoir réducteur procuré par une fonction hémiacétalique ou cétonique.

Cette réaction de dégradation alcaline oxydative se traduit par la formation, à partir du résidu porteur de cette fonction hémiacétalique ou cétonique, d'un résidu ayant perdu un atome de carbone mais qui devient porteur d'une fonction carboxylique.

De l'acide formique se forme de façon concomitante à partir de cet atome de carbone soustrait au résidu porteur de la fonction hémiacétalique ou cétonique réductrice.

Dans cette première étape de dégradation alcaline oxydative selon le procédé conforme à l'invention, les divers composants des hydrolysats d'amidon, à savoir le glucose, le maltose, les oligoglucosyl-glucose et les polyglucosyl-glucose sont donc transformés respectivement en arabinonate, glucosyl-arabinonate, oligoglucosyl-arabinonate et polyglucosyl-arabinonate avec formation simultanée de formiate.

Cette réaction de dégradation oxydative alcaline faisant apparaître au moins deux molécules d'acide (une molécule d'arabinonate ou de glucosyl-arabinonate et une molécule de formiate) par molécule de sucre réducteur mise en oeuvre, il est nécessaire, soit de prévoir une réserve alcaline suffisante pour mener la réaction à son terme, soit d'ajouter l'alcali au fur et à mesure de sa consommation par la réaction.

Généralement, il convient d'utiliser de 2,1 à 3 moles et de préférence de 2,2 à 2,4 moles de soude ou de potasse par mole de fonction hémiacétalique ou cétonique à oxyder, mais d'autres alcalis employés dans ces mêmes proportions peuvent aussi convenir.

La concentration des hydrolysats d'amidon soumis à cette étape d'oxydation importe peu pourvu que l'on dispose de réacteurs dotés de moyens d'agitation et d'aération efficaces. On préférera toutefois procéder à l'étape de dégradation alcaline oxydative sur des solutions aqueuses d'hydrolysat d'amidon d'une concentration de 10 à 60 % et de préférence, de 25 à 40 % de matières sèches.

Cette étape d'oxydation étant fortement exothermique, il convient d'employer des réacteurs munis d'un dispositif de refroidissement efficace. On préfère effectuer cette étape d'oxydation à une température comprise entre 20 et 70 °C et de préférence entre 25 et 65°C.

Il faut noter qu'en règle générale, les températures les plus basses permettent d'obtenir les sélectivités les meilleures mais que ceci a lieu au détriment de la vitesse de réaction. A l'inverse, les températures plus élevées permettent de raccourcir les temps de réaction et peuvent être employées dans la mesure où une légère dépolymérisation de l'hydrolysat d'amidon ainsi que l'obtention de quelques pour-cent d'acides carbonique, oxalique, glycérique, glycolique, lactique, érythronique, métasaccharinique, dihydroxybutyrique ne sont pas préjudiciables au déroulement correct de la suite du procédé ni à l'utilisation des produits obtenus pour la confection de compositions lessivielles.

Que l'on travaille ou non en présence de catalyseurs d'oxydation (bleu de méthylène, acide anthraquinone-2-monosulfonique, eau oxygénée, on préfère laisser se poursuivre la réaction jusqu'à l'obtention d'une teneur en sucres réducteurs, mesurée par la méthode de BERTRAND, comprise entre 0,1 et 2 %, de préférence comprise entre 0,2 et 1 %, cette teneur étant exprimée en pourcentage pondéral d'équivalent glucose par rapport à la matière sèche du contenu du réacteur.

On notera qu'il serait déraisonnable de prolonger la réaction au-delà de ce seuil de sucres réducteurs puisque l'étape suivante permettra encore de l'abaisser.

C'est d'ailleurs un des gros avantages du procédé de l'invention, que de fournir des produits dont la teneur en sucres réducteurs se trouve amenée à une valeur si basse qu'ils ne montrent quasiment plus aucune sensibilité à la chaleur ou aux milieux alcalins et ce même après des périodes de stockage prolongées.

Au terme de cette réaction de dégradation alcaline oxydative, on procède, s'il en est besoin, à l'enlèvement du catalyseur par percolation du milieu réactionnel sur une colonne de charbon actif par exemple.

La seconde étape du procédé selon l'invention consiste à oxyder sélectivement tout ou partie des fonctions alcools primaires des glucosyl-arabinonates obtenus à l'étape précédente. Elle peut être conduite de diverses manières.

On peut employer, par exemple, les procédés d'oxydation à l'acide nitrique, au dioxyde ou tétroxyde d'azote tels que déjà cités. Toutefois, ces procédés obligent à neutraliser l'excédent d'alcali nécessaire à la réalisation de la première étape et ils obligent également à sécher le produit obtenu après cette première étape.

On préfère donc procéder à cette oxydation des fonctions alcools primaires par une méthode efficace en milieu alcalin et qui puisse donc tirer profit de l'excédent d'alcali utilisé lors de la première étape. De même, comme on préfère ne pas devoir procéder à un séchage intermédiaire du glucosyl-arabinonate, on préfère employer une méthode efficace sur les solutions aqueuses de glucosyl-arabinonates.

Une méthode d'oxydation particulièrement préférée selon le procédé conforme à l'invention est celle qui est décrite dans la demande internationale de brevet WO 95/07303 et qui permet l'obtention d'acides poly-α-glucuroniques à partir d'hydrolysats d'amidon ou d'inuline.

Dans le procédé selon l'invention, on soumet donc les glucosyl-arabinonates obtenus lors de l'étape précédente à l'action d'un hypohalite en présence d'une quantité catalytique d'un cation nitroxyl alcyle binaire ou tertiaire. Comme cette réaction d'oxydation se déroule au mieux à un pH compris entre 9 et 13, on tire ainsi aisément parti de l'excès d'alcali nécessaire à l'accomplissement de l'étape d'oxydation précédente.

Comme dans la demande de brevet précitée, on préfère utiliser comme catalyseur d'oxydation la 1-oxyl-2,2,6,6-tétraméthylpipéridine, ci-après désignée par TEMPO.

Dans cette réaction d'oxydation, l'oxydant réel est le cation nitrosonium qui est réduit en hydroxylamine lorsqu'une fonction alcool primaire est oxydée en fonction acide carboxylique. Ce cation nitrosonium est régénéré in situ par un oxydant qui est constitué le plus commodément par un couple hypochlorite/bromure. Lors de la réaction, on maintient un pH constant par l'addition d'une base qui est de préférence la même que celle qui a servi lors de la première étape d'oxydation.

Tout ou partie des motifs anhydroglucosyl des glucosyl-arabinonates sont ainsi oxydés en motifs anhydroglucuronyl et l'acide arabinonique terminal est ainsi oxydé en acide arabinarique.

Cette réaction d'oxydation des fonctions alcools primaires s'effectue d'après cette demande de brevet WO 95/07303 à une température inférieure à 30°C, de préférence comprise entre 0 et 5°C, et à une concentration en matières sèches d'environ 7 à 15 grammes par litre d'eau, ainsi qu'il est indiqué dans la demande de brevet WO 95/07303.

La Société Demanderesse a toutefois remarqué que l'on pouvait, sans inconvénient, utiliser des concentrations beaucoup plus élevées et des températures également plus élevées allant jusqu'à 50°C, ce qui se traduit par des volumes de réacteur plus faibles et la possibilité de s'affranchir de l'utilisation de groupes frigorifiques.

Le TEMPO est ajouté à raison de 0,1 à 2,5 % en poids par rapport au poids de glucosyl-arabinonate à oxyder en glucuronyl-arabinarate.

L'hypochlorite de sodium est mis en oeuvre à raison de 2 moles par mole d'alcool primaire à oxyder.

Dans la pratique, on préfère cependant utiliser jusqu'à 10 % de NaOCl en excès par rapport à la stoechiométrie de la réaction.

L'hypochlorite de sodium est donc mis en oeuvre en tenant compte de cet excès, de préférence à raison de 1,1 à 2,2 moles par mole d'alcool primaire, selon le degré d'oxydation souhaité qui peut varier de 50 à 100 %.

Comme co-oxydant, on peut ajouter du bromure de sodium à raison de 0,1 à 1 mole et de préférence à raison de 0,2 à 0,5 mole par mole de NaOCl mise en oeuvre afin d'accélérer la réaction d'oxydation.

L'oxydation est généralement complète au bout de 1 heure. A ce stade, la teneur en sucres réducteurs du milieu réactionnel a encore chuté et est devenue généralement inférieure à 0,5 %, plus généralement encore à 0,2 %.

On procède ensuite à l'extraction par l'éther du milieu réactionnel, ou mieux à sa percolation sur une colonne de charbon activé granulaire pour en ôter le TEMPO.

Après filtration du milieu réactionnel purifié, on procède généralement à sa concentration jusqu'à une matière sèche de 20 % puis éventuellement à sa déshydratation si l'on désire que les produits de l'invention entrent dans la composition de lessives se présentant sous forme de poudre. Une telle déshydratation n'est évidemment pas nécessaire dans la mesure où l'on désire formuler des lessives liquides.

On peut aussi, si on le désire, après concentration mais avant séchage, procéder à l'élimination du chlorure de sodium qui se forme au cours de la deuxième étape du procédé ainsi qu'à celle du bromure de sodium que l'on a éventuellement ajouté comme co-oxydant, par des techniques connues de l'homme de l'art telles que la chromatographie d'exclusion d'ions sur résines cationiques fortes.

L'exemple qui suit a pour objet d'illustrer et de mieux faire comprendre l'invention.

### Exemple 1 :

### 1ère étape : dégradation alcaline oxydative d'un hydrolysat d'amidon

Dans un fermenteur à cuve de verre de marque BIOLAFITTE, d'une contenance utile de 20 litres, on introduit 2995 grammes d'eau et 1591 grammes de soude pour former 4586 grammes d'une lessive de soude à 34,7 %.

On ajoute alors dans cette lessive, 55 grammes d'anthraquinone-2-monosulfonate de sodium et 18,2 ml d'eau oxygénée à 110 volumes.

On aère alors le fermenteur avec un débit d'air de 20 litres par minute et en l'agitant à la vitesse de 1000 tours par minute.

Après avoir agité ce mélange à 25°C durant 30 minutes, on porte la température à 45°C. On ajoute alors lentement et de façon régulière, en 3 heures 30 minutes, 18.344 grammes d'un sirop de glucose obtenu par hydrolyse acide d'amidon de maïs, présentant une matière sèche de 50 % et un DE de 37 (soit un degré de polymérisation moyen égal à 2,7).

On fixe ensuite la température à 55°C et l'on poursuit l'agitation et l'aération durant 2 heures 30 minutes. La teneur en sucres réducteurs du milieu réactionnel s'est alors abaissée à 0,3 g/ 100 g de matières séches de sirop de glucose. (Elle était au départ de 37 g/ 100 g)

Le milieu réactionnel est ensuite percolé sur une colonne de charbon activé granulaire de manière à en ôter le sel de sodium de l'acide-2-anthraquinone monosulfonique.

### 2ème étape : oxydation sélective des fonctions alcools primaires du glycosyl arabinonate

Dans une cuve agitée d'un volume total de 35 litres contenant 8 litres d'eau, on ajoute 1146 grammes de la solution obtenue à l'étape précédente (ce qui correspond à 459 g de matière sèche de sirop de glucose mis en oeuvre à l'étape précédente) et on refroidit le tout à 5°C.

On additionne alors 42,7 grammes de bromure de sodium et 4,17 grammes de TEMPO. Après avoir laissé la dissolution de ces ingrédients se faire, ce qui prend quelques minutes, on ajoute en une seule fois 2,9 litres d'une solution d'hypochlorite de sodium à 157 g/l (eau de javel à 48° chlorométriques) préalablement diluée à 25 % et ajustée à pH 10,4 par de l'acide chlorhydrique.

On maintient alors la température à 5°C par addition d'un peu de glace et le pH à 10,4 par addition continue de soude à 10 %.

Après 60 minutes, la consommation de soude est devenue nulle, indiquant la fin de la réaction.

On procède alors à l'enlèvement du TEMPO par percolation du milieu réactionnel sur une colonne de charbon activé granulaire puis l'on concentre le milieu réactionnel purifié, après l'avoir filtré, jusqu'à une concentration de 20 % de matières sèches.

Le glucuronyl-arabinarate brut ainsi obtenu a révélé l'analyse suivante, les pourcentages étant exprimés sur la matière sèche du milieu réactionnel concentré :
- Produits de dégradation
   (oxalate, glycérate, glycolate, lactate...) 1 %
- Matière active
   (glucuronyl-arabinarates) 39,6 %
- NaCl 51,5 %
- NaBr 3,1 %
- Formiate 4,8 %
- Taux de carboxylate de sodium
   (en poids % matière active) 17,5 %
Soit un taux de conversion proche de 100 %
- Taux de sucres réducteurs
   (sur matière sèche totale) 0,15 %
   (sur matière active) 0,38 %
- Degré de polymérisation (DP)
   moyen de la matière active 2,7

### Exemple 2 :

On a procédé à l'enrichissement en matières actives du produit obtenu à l'exemple 1 par une technique chromatographique d'exclusion d'ions sur résines cationiques fortes.

On a ainsi obtenu un produit ne titrant plus que 5% de chlorure de sodium, que l'on a atomisé pour obtenir une poudre blanche.

On a utilisé cette poudre en substitution des polyacrylates dans une formule lessivielle à raison de 1 partie de glucuronyl-arabinarates ainsi enrichis pour 1 partie de polyacrylates.

Non seulement les poudres obtenues ne colorent pas au stockage, mais elles présentent des qualités lessivielles très intéressantes puisqu'après avoir effectué 25 lavages consécutifs d'échantillons de tissus de coton et de coton/polyester, les indices de blancheur obtenus s'avèrent supérieurs au témoin polyacrylate.

En outre, le taux d'incrustations organiques s'avère significativement inférieur.

## Revendications

1. Glucuronyl-arabinarates.

2. Compositions de glucuronyl-arabinarates ayant un degré de polymérisation moyen égal ou supérieur à 2.

3. Compositions de glucuronyl-arabinarates ayant un degré de polymérisation moyen compris entre 2 et 50 et de préférence compris entre 2 et 10.

4. Compositions de glucuronyl-arabinarates selon l'une quelconque des revendications 1 à 3, caractérisées par le fait qu'elles contiennent de 100 % à 50 %, de préférence de 99 à 60 %, de motifs anhydroglucuronyl.

5. Compositions de glucuronyl-arabinarates selon la revendication 4, caractérisées par le fait qu'elles contiennent moins de 1 %, de préférence moins de 0,7 %, et plus préférentiellement encore moins de 0,5 % de sucres réducteurs libres.

6. Procédé de fabrication de glucuronyl-arabinarates, caractérisé par le fait que :
- dans une première étape, on procède à la dégradation alcaline oxydative d'un hydrolysat d'amidon de manière à obtenir des glucosyl-arabinonates,
- dans une deuxième étape, on procède à l'oxydation sélective de tout ou partie des fonctions alcools primaires de ces glucosyl-arabinonates.

7. Procédé de fabrication de glucuronyl-arabinarates selon la revendication 6, caractérisé par le fait que la deuxième étape est effectuée par action d'un hypohalite en présence d'une quantité catalytique d'un cation nitroxyl alcyle binaire ou tertiaire.

8. Procédé de fabrication de glucuronyl-arabinarates selon la revendication 7, caractérisé par le fait que le cation nitroxyl alcyle binaire ou tertiaire est le cation nitrosonium.

9. Procédé de fabrication de glucuronyl-arabinarates. selon l'une des revendications 7 et 8, caractérisé par le fait que l'hypohalite est l'hypochlorite de sodium et qu'il est mis en oeuvre à raison de 1,1 à 2,2 moles par mole d'alcool primaire à oxyder.

10. Utilisation de glucuronyl-arabinarates pour la formulation de compositions lessivielles.

## Patentansprüche

1. Glucuronyl-arabinarate.

2. Glucuronyl-arabinarate-Zusammensetzungen mit einem mittleren Polymerisationsgrad gleich oder über 2.

3. Glucuronyl-arabinarate-Zusammensetzungen mit einem mittleren Polymerisationsgrad zwischen 2 und 50 und vorzugsweise zwischen 2 und 10.

4. Glucuronyl-arabinarate-Zusammensetzungen nach einem der Ansprüche 1 bis 3, dadurch gekennzeichnet, dass sie 100 bis 50 %, vorzugsweise 99 bis 60 %, Anhydroglucuronyleinheiten enthalten.

5. Glucuronyl-arabinarate-Zusammensetzungen nach Anspruch 4, dadurch gekennzeichnet, dass sie weniger als 1 %, vorzugweise weniger als 0,7 % und insbesondere weniger als 0,5 % freie reduzierende Zucker enthalten.

6. Verfahren zur Herstellung von Glucuronyl-arabinaraten, dadurch gekennzeichnet, dass man
- in einem ersten Schritt den oxidativen alkalischen Abbau eines Stärkehydrolysats so vornimmt, dass man Glucosyl-arabinonate erhält, und
- in einem zweiten Schritt die selektive Oxidation der primären Alkoholgruppen dieser Glucosyl-arabinonate ganz oder teilweise vornimmt.

7. Verfahren zur Herstellung von Glucuronyl-arabinaraten nach Anspruch 6, dadurch gekennzeichnet, dass der zweite Schritt durch Einwirkung eines Hypohalogenits in Gegenwart einer katalytischen Menge eines binären oder tertiären Nitroxylalkyl-Kations durchgeführt wird.

8. Verfahren zur Herstellung von Glucuronyl-arabinaraten nach Anspruch 7, dadurch gekennzeichnet, dass das binäre oder tertiäre Nitroxylalkyl-Kation das Nitrosonium-Kation ist.

9. Verfahren zur Herstellung von Glucuronyl-arabinaraten nach einem der Ansprüche 7 und 8, dadurch gekennzeichnet, dass das Hypohalogenit Natriumhypochlorit ist und dass es in einer Menge von 1,1 bis 2,2 Mol pro Mol zu oxidierender primärer Alkohol verwendet wird.

10. Verwendung von Glucuronyl-arabinaraten für die Formulierung von Waschmittelzusammensetzungen.

## Claims

1. Glucuronyl arabinarates.

2. Compositions of glucuronyl arabinarates having a mean degree of polymerization greater than or equal to 2.

3. Compositions of glucuronyl arabinarates having a mean degree of polymerization of between 2 and 50 and preferably of between 2 and 10.

4. Compositions of glucuronyl arabinarates according to any one of Claims 1 to 3, characterized in that they contain from 100 % to 50 %, preferably from 99 to 60 %, of anhydroglucuronyl units.

5. Compositions of glucuronyl arabinarates according to Claim 4, characterized in that they contain less than 1 %, preferably less than 0.7 %, and still more preferably less than 0.5 % of free reducing sugars.

6. Process for the manufacture of glucuronyl arabinarates, characterized in that:
- in a first stage, the oxidative alkaline degradation of a starch hydrolysate is carried out so as to obtain glucosyl arabinonates,
- in a second stage, the selective oxidation of all or part of the primary alcohol functional groups of these glucosyl arabinonates is carried out.

7. Process for the manufacture of glucuronyl arabinarates according to Claim 6, characterized in that the second stage is carried out by action of a hypohalite in the presence of a catalytic amount of a binary or tertiary alkyl nitroxyl cation.

8. Process for the manufacture of glucuronyl arabinarates according to Claim 7, characterized in that the binary or tertiary alkyl nitroxyl cation is the nitrosonium cation.

9. Process for the manufacture of glucuronyl arabinarates according to one of Claims 7 and 8, characterized in that the hypohalite is sodium hypochlorite and in that it is used in an amount of 1.1 to 2.2 mol per mole of primary alcohol to be oxidized.

10. Use of glucuronyl arabinarates for the formulation of detergent compositions.
